(19) Europäisches Patentamt
European Patent Office
Office européen des brevets

(11) **EP 3 320 830 A1**

(12) **EUROPEAN PATENT APPLICATION**

(43) Date of publication:
**16.05.2018 Bulletin 2018/20**

(51) Int Cl.:
***A61B 3/107*** (2006.01)     ***A61B 3/00*** (2006.01)
***G06F 19/00*** (2018.01)

(21) Application number: **16198190.7**

(22) Date of filing: **10.11.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **IROC Science AG**
**8005 Zürich (CH)**

(72) Inventors:
• **Mrochen, Michael**
**8193 Eglisau (CH)**
• **Boss, Daniel**
**8048 Zürich (CH)**

(74) Representative: **Frenkel, Matthias Alexander**
**Wuesthoff & Wuesthoff**
**Patentanwälte PartG mbB**
**Schweigerstrasse 2**
**81541 München (DE)**

(54) **TECHNIQUE FOR PERFORMING OPHTHALMIC MEASUREMENTS ON AN EYE**

(57)     A device for performing ophthalmic measurements on an eye is presented. The device comprises a plurality of first light sources each configured to emit light towards a cornea of the eye and a plurality of first optical detectors each configured to generate a two-dimensional image of a plurality of light spots each resulting from light emitted by one of the plurality of first light sources and reflected by the cornea towards the corresponding first optical detector. The device further comprises a controller configured to determine topographic features of the cornea and a position of the eye with respect to the device by performing raytracing on a modelled optical configuration and by comparing results of the raytracing with positions of the plurality of first light sources and/or with positions of the light spots in the two-dimensional images. Further, a method for performing ophthalmic measurements on an eye is presented.

Fig. 1

EP 3 320 830 A1

**Description**

**Technical Field**

The present disclosure relates to a technique for performing ophthalmic measurements on an eye. The technique may be embodied in at least one device and/or at least one method.

**Background**

There are many situations in which it is desirable to achieve precise ophthalmic measurement results, wherein such ophthalmic measurements may include measurements of a topography of the cornea of the eye (in particular, of the anterior cornea), measurements of aberrations of the eye, wherein it is particularly desirable to obtain information on higher order aberrations of the eye, and measurements of intraocular distances, such as axial length measurements, wherein distances between intraocular surfaces of the eye are determined.

One such situation, for instance, relates to the progression of myopia in children, for which evidence of an "epidemic of myopia", especially in Asian countries and/or urban environments has accumulated. In order to diagnose myopic progression and provide a control for treatments aimed at slowing/or stopping myopic progression it would be desirable to monitor axial length, refraction, corneal curvature, and/or higher order corneal and ocular aberrations over time.

Currently, routine examinations require a visit to an ophthalmic clinic, where adequate instrumentation are available to perform the aforementioned ophthalmic measurements. It would be desirable to have more flexibility by providing handheld portable solutions for performing the aforementioned measurements.

In other situations, these measurements can be used, e.g., to decide whether an eye is healthy or not (e.g., suffers from keratoconus). Further, the measurements may be used to fit a contact lens onto the eye or to decide whether a patient needs a corrective lens or not. Further, parameters necessary for producing a correction lens for the measured eye may be obtained from such measurements. Further, in cataract surgery, postoperative measurements would allow a cataract surgeon to optimize intra-ocular lens selections and in refractive laser procedures to adjust the treatment selection by using postoperative objective refraction measurements and statistical analysis (cf. "Nomogram computation and application system and method for refractive laser surgery", US 8403919 B2).

Handheld, non-contact measurements have a problem of not allowing for accurate control of the eye position with respect to the measurement apparatus. Uncertainty in the eye position can affect accuracy of the measurement of the eye parameters. It would therefore be beneficial to render the measurement of the eye parameter with the handheld apparatus independent of the relative eye position.

Although devices are known which measure one or more of the aforementioned parameters of an eye, an accuracy, a range of functions and/or a flexibility of these prior art devices still needs to be improved.

**Summary**

It is therefore an object of the present disclosure to provide a technique for performing ophthalmic measurements on an eye, which avoids one or more of the drawbacks discussed above, or other related problems.

According to a first aspect, a device for performing ophthalmic measurements on an eye is provided. The device comprises a plurality of first light sources each configured to emit light towards a cornea of the eye and a plurality of first optical detectors each configured to generate a two-dimensional image of a plurality of light spots each resulting from light emitted by one of the plurality of first light sources and reflected by the cornea towards the corresponding first optical detector. The device further comprises a controller configured to determine topographic features of the cornea and a position of the eye with respect to the device by performing raytracing on a modelled optical configuration and by comparing results of the raytracing with positions of the plurality of first light sources and/or with positions of the light spots in the two-dimensional images.

Although in the following, details with regard to the first aspect are discussed, these details also hold for the second aspect described in this disclosure, where applicable.

In this disclosure, the expressions "first", "second", "third", etc. are provided merely for distinguishing between different elements and/or entities. These expressions are not limiting with regard to an arrangement or any other properties of the respective element or entity. For example, the expression "first optical detector" is merely used to enable distinction from a "second optical detector" and a "third optical detector", and so forth. The expression "first" in "first optical detector" does not imply any properties of this optical detector, such as an arrangement within the device or a detection wavelength or the like.

The eye may be an eye of a human patient or of an animal. The device may be a handheld device configured to be held by an operator during the measurements with one or both hands. The device may be an autonomous handheld device without the necessity to be connected to an external power source and/or control device. Further, the device may be configured such that measurements can be carried out when the patient is in an upright (e.g. standing or sitting) position and/or when the patient is in a surgical position, such as a supine position (i.e. lying on his/her back). The plurality of first light sources may each emit light having a wavelength in a range between 320 and 1500 nm. The first optical detectors may each comprise, e.g., a CCD sensor or a CMOS sensor for generating a corresponding two-dimensional image. Further, each of the first optical detectors may comprise corresponding image generating optics comprising, e.g., a converging lens. Further, zooming optics and/or focusing optics may be provided for changing a focal length and/or a position of an image plane for the corresponding first optical detector.

The light spots detected by each of the first optical detectors may result from light which is emitted by the plurality of first light sources and reflected by specular reflection on an exterior surface of the cornea of the eye. Thus, each light spot may be associated with one of the plurality of first light sources.

Raytracing may be carried out using known raytracing algorithms e.g. in a three-dimensional space. The modelled optical configuration may correspond to an "imaginary" configuration which is used by the controller for the raytracing. The modelled optical configuration may be supposed to represent the "real" optical configuration as close as possible. A predefined initial modelled optical configuration may be used as a starting point for the raytracing. The topographic features of the cornea and a position of the cornea with respect to the device may be determined by adjusting the modelled optical configuration (including a model cornea) until a result of the raytracing represents the result of the measurement as closely as possible. For example, the modelled optical configuration may be adjusted for a predetermined number of times (e.g., N=2) or until a difference between the raytracing result and the measurement result is below a predefined threshold.

The controller may be configured to determine topographic features of the cornea and a position of the eye with respect to the device by performing raytracing on a modelled optical configuration and by comparing results of the raytracing with positions of the plurality of first light sources and/or with positions of the light spots in at least one, in at least two, or in all of the two-dimensional images, for example. In order to precisely determine the position of the eye with respect to the device, it may be advantageous to analyze at least two (or any arbitrary number larger than two) of the two-dimensional images.

By using the above raytracing technique, it may be possible to reconstruct topographic features of the cornea under consideration of the reconstructed position of the eye. This is possible even when the eye is not centered with regard to an optical axis of the device and/or when the eye has an arbitrary distance from the device.

In this disclosure, a "position of a light spot" may correspond to a two-dimensional centroid position of said light spot, which may be determined by known algorithms. Just as a simple example, the position of the light spot may be determined to correspond to the pixel having the highest brightness value within a predefined area on a corresponding two-dimensional image sensor (e.g. CCD or CMOS). Further, methods may be applied, in which the centroid is determined by more complex algorithms.

The controller may be configured to consider a model cornea as part of the modelled optical configuration, perform raytracing of light rays emitted by model light sources and reflected by the model cornea, compare results of the raytracing with positions of the plurality of first light sources and/or with positions of the light spots in at least one of the two-dimensional images, adjust topographic features of the model cornea and/or a position of the model cornea, repeat the steps of considering, performing, and comparing, and determine the topographic features of the cornea and the position of the eye based on the topographic features of the model cornea and the position of the model cornea.

For example, the topographic features of the cornea and the position of the eye may be determined so as to correspond to the topographic features of the model cornea and the position of the model cornea, respectively. In other words, the position of the eye may correspond to the position of the cornea of the eye. Further, the position of the eye may be derived from the position of the model cornea.

Further, the steps of considering, performing, and comparing may be carried out for each two-dimensional image generated by the plurality of first optical detectors. Alternatively, the steps of considering, performing, and comparing may be carried out for at least two of the two-dimensional images.

The topographic features and/or the position may be determined as a mean value of topographic features and positions of the model cornea resulting from raytracing procedures for each first optical detector. Further, the steps of considering, performing, comparing, and adjusting may be performed a plurality of times in an iterative process. In the step of comparing, distance values may be generated. These distance values may each correspond to a distance between a measured light spot and a light spot resulting from the raytracing. Further, the distance values may each correspond to a distance between a position of one of the first light sources and a position of a model light source resulting from backwards raytracing based on the measured light spots.

The controller may be configured to perform the raytracing by raytracing of light rays emitted at known positions of the plurality of first light sources and compare positions of the raytraced light rays on a model optical detector with measured positions of the light spots in at least one of the two-dimensional images.

This technique may also be referred to as "forward raytracing".

The step of adapting may be carried out so as to reduce differences between the positions of the raytraced light rays on the model optical detector and the measured positions of the light spots in at least one of the two-dimensional images. For example, an iterative process may be carried out in which the steps of considering, performing, comparing, and adjusting are carried out until a distance measure is below a predefined threshold value. The distance measure may correspond to a minimum value, a maximum value, or an average value of the differences between the positions of the raytraced light rays on the model optical detector and the measured positions of the light spots for one of the two-dimensional images.

The controller may be configured to perform the raytracing by raytracing of light rays back from measured positions of the light spots towards the model cornea and compare positions of model light sources determined by the ray tracing with known positions of the first light sources.

This technique may also be referred to as "backward raytracing".

The positions of the model light sources may be determined as points of intersection between raytraced light rays and a predefined model light source plane. The step of adapting may be carried out so as to reduce differences between the positions of the model light sources determined by the raytracing and the known positions of the first light sources. For example, an iterative process may be carried out in which the steps of considering, performing, comparing, and adjusting are carried out until a distance measure is below a predefined threshold value. The distance measure may correspond to a minimum value, a maximum value, or an average value of the differences between the positions of the model light sources determined by the raytracing and the known positions of the first light sources.

The plurality of first light sources may be arranged at predetermined positions with regard to a center axis of the device. Each of the plurality of first optical detectors may be arranged at a location away from the center axis of the device.

The plurality of first light sources may comprise light sources arranged along a first circle, wherein an axis extending through the center of the first circle and being orthogonal to the first circle may be defined as the center axis of the device. Further, the plurality of first light sources may comprise light sources arranged along a second circle having the same center as the first circle and a different radius than the first circle.

The plurality of first optical detectors may be located on the first circle or on the second circle, for example.

The topographic features of the cornea may comprise at least one feature selected from the list of radius along a steep axis, curvature along a steep axis, asphericity along a steep axis, radius along a flat axis, curvature along a flat axis, asphericity along a flat axis, orientation of a steep axis with regard to a reference axis of the eye, orientation of a flat axis with regard to a reference axis of the eye, radius along a horizontal axis, curvature along a horizontal axis, asphericity along a horizontal axis, radius along a vertical axis, curvature along a vertical axis, asphericity along a vertical axis, and higher order corneal aberrations. The position of the eye with respect to the device may be represented by coordinates of the intersection of a reference axis of the eye and the anterior corneal surface of the eye with respect to a coordinate system fixed to the device.

The position of the eye with respect to the device may be represented by coordinates of a corneal vertex of the eye with respect to the coordinate system fixed to the device. The corneal vertex may be defined as the most anterior point of the cornea when the patient is fixating a target lying on the center axis of the device. The coordinate system fixed to the device may also be referred to as a device anchored coordinate system.

The device may further comprise a second light source for illuminating the eye with light so as to produce a wavefront that propagates along an optical path and a wavefront sensor configured to provide a measure indicative of aberrations of the eye.

The second light source may emit light having a wavelength in a range between 320 and 1500 nm. The second light source may comprise a laser configured to illuminate the eye such that an illumination spot is generated on the retina of the eye. The illumination spot may serve as a point source for generating the wavefront. The wavefront may be influenced by features of the eye, such as the lens of the eye, the vitreous body of the eye, the cornea of the eye, etc. The optical path may correspond to a line of sight of the eye and/or to a center axis of the device.

The controller may be configured to determine wavefront aberrations with regard to a first plane, wherein the first plane has a fixed distance from the device, and determine wavefront aberrations of the eye with regard to a second plane having a predetermined position with regard to the position of the eye by backpropagating the determined wavefront from the first plane to the second plane, based on the measured position of the eye with respect to the device.

The first plane may be predefined by an optical configuration of the device. A first relay lens and a second relay lens may be provided for imaging the first plane onto a plane of the lenslet array. In other words, the first plane and a plane in which the lenslet array are located may be optically conjugate planes. The backpropagating may be carried out using known algorithms, for instance the method of angular spectrum propagation as described in Goodman, J. W. (1996) "Introduction to Fourier Optics", McGraw-Hill Series in Electrical and Computer Engineering. The second plane may correspond to a pupil plane of the eye or to a spectacle plane in a fixed distance in front of the eye.

The wavefront sensor may comprise a two-dimensional lenslet array and a second optical detector for generating a two-dimensional image of light spots generated by lenslets of the lenslet array by focusing the wavefront onto the second optical detector.

The wavefront sensor may comprise a Hartman-Shack sensor (also referred to as a Shack-Hartman sensor or Shack-Hartman wavefront sensor, SHWFS). The aberrations of the eye (e.g., low order aberrations and/or high order aberrations) may be determined by the Hartman-Shack sensor in a known manner. In particular, differences between known light spot positions generated on the second optical detector by a plane wave and actual light spot positions generated by the wavefront may be compared. Based on the comparison, e.g., the aberrations may be expressed in terms of Zernike polynomials.

The controller may be configured to determine an average eye orientation with respect to a device anchored coordinate system from a set of topographic measurements obtained from the first optical detectors, and align the set of topographic measurements with respect to the average eye orientation by means of evaluated alignment transformations.

Here, the expression "topographic measurements" refers to individual topography images from which the eye position can be determined.

The controller may be configured to align a set of wavefront measurements performed by the wavefront sensor with respect to an eye anchored coordinate system by means of said alignment transformations.

The controller may be configured to calculate internal aberrations of the eye by subtracting corneal aberrations of the eye from total aberrations of the eye, wherein the corneal aberrations are based on the determined topographic features of the eye and the total aberrations are based on the measure indicative of aberrations of the eye provided by the wavefront sensor.

The controller may be configured to determine the corneal aberrations $W_c(x,y)$ by using the following equation: $W_c(x,y) = (n_c-1)*z_c(x,y)*2*\pi/\lambda$, where x and y represent coordinates with regard to a plane perpendicular to the line of sight of the eye, $n_c$ represents a refractive index of the cornea of the eye, $z_c$ represents a height profile of the cornea, and $\lambda$ represents a wavelength of the second light source.

The device may further comprise an optical coherence tomography unit for determining intra-ocular distances comprising a light coupler, a third light source configured to emit light towards the light coupler, a reference arm comprising an adjustable reference mirror, an object arm configured to generate an object beam for directing light generated by the third light source towards the eye, and a detector arm comprising a third optical detector. The light coupler is configured to couple part of the light generated by the third light source into the reference arm and to couple part of the light generated by the third light source into the object arm. The light coupler is further configured to couple light reflected by the eye into the detector arm and to couple light reflected by the adjustable reference mirror into the detector arm, such that the light reflected by the eye and the light reflected by the adjustable reference mirror interfere at the third optical detector.

The light coupler may comprise a beam splitter. Each of the reference arm, the object arm, and the detector arm may comprise an optical fiber. Further, the light emitted by the third light source may be directed to the light coupler via an optical fiber. The optical coherence tomography unit may be configured to measure intraocular distances within the eye. In other words, the optical coherence tomography unit may be configured to measure intraocular positions of surfaces in the eye. The optical coherence tomography unit may be configured to measure positions of intraocular surfaces with regard to a coordinate system fixed to the device. Further, the determined position of the cornea with respect to the device may be used for calculating an intraocular distance between the cornea and the respective intraocular surface. For example, a distance between an apex position of the cornea and the retina of the eye may be determined.

The third light source of the optical coherence tomography unit may serve as the second light source.

A semi-transparent mirror may be used in order to direct the light emitted by the third light source towards the eye. The semi-transparent mirror may be configured as a beam splitter, wherein part of the light reflected by the eye is transmitted towards the wavefront sensor and part of the light reflected by the eye is reflected towards the optical coherence tomography unit. More precisely, the reflected part may be reflected towards the object arm of the optical coherence tomography unit. From the object arm, the light reflected by the eye may be guided to the detector arm of the optical coherence tomography unit.

The controller may be configured to set an initial position of the adjustable reference mirror based on the determined position of the eye. Additionally or alternatively, the device may comprise a tunable lens within the object beam and the controller may be configured to set an initial focal length of the tunable lens based on the determined position of the eye.

According to a second aspect, a method for performing ophthalmic measurements on an eye with a device is provided. The method comprises emitting light towards a cornea of the eye by a plurality of first light sources of the device and generating, by a plurality of first optical detectors of the device, a plurality of two-dimensional images of a plurality of light spots each resulting from light emitted by one of the plurality of first light sources and reflected by the cornea towards the corresponding first optical detector. The method further comprises determining topographic features of the cornea and a position of the eye with respect to the device by performing raytracing on a modelled optical configuration and by comparing results of the raytracing with positions of the plurality of first light sources and/or with positions of the light spots in the two-dimensional images.

The method may be carried out by a device described in the present disclosure, e.g., by the device according to the first aspect.

**Brief Description of the Drawings**

Embodiments of the technique presented herein are described below with reference to the accompanying drawings, in which:

Fig. 1       shows a schematic cross-sectional side view of a basic configuration of a device for performing ophthalmic measurements on an eye according to the present disclosure;

Fig. 2       shows a schematic cross-sectional front view of a basic configuration of a device according to the present disclosure;

Fig. 3a      shows an exemplary schematic representation of a two-dimensional image generated by a first one of a plurality of first optical detectors of a device according to the present disclosure;

Fig. 3b      shows an exemplary schematic representation of a two-dimensional image generated by a second one of a plurality of first optical detectors of a device according to the present disclosure;

Fig. 3c      shows an exemplary schematic representation of a two-dimensional image generated by a third one of a plurality of first optical detectors of a device according to the present disclosure;

Fig. 4a      shows a schematic representation of a device incorporating means for measuring and displaying an orientation of the device;

Fig. 4b      shows a schematic representation of a device having means for determining a reference axis of an eye of the patient in an upright position based on an orientation sensor;

Fig. 5a      shows a schematic representation of a device having means for capturing an image with a wide field of view;

Fig. 5b      shows a schematic representation of a device having means for determining a reference axis of an eye based on an image of the patient's face;

Fig. 6a      shows a schematic representation of a device aligned with an eye axis of a patient;

Fig. 6b      shows a schematic representation of a device tilted with respect to an eye axis of a patient;

Fig. 7       shows exemplary modelled raytracing spot positions as circles and exemplary detected spot positions as crosses, for one of the first optical detectors;

Fig. 8       shows a flowchart of a method for determining topographic features of the cornea and a position of the cornea with respect to a device according to the present disclosure;

Fig. 9       shows a schematic cross-sectional side view of a device for performing ophthalmic measurements on an eye according to the present disclosure, wherein the device comprises a topography unit and a wavefront unit;

Fig. 10a     shows a schematic representation of a device aligned with an eye axis of a patient;

Fig. 10b     shows a schematic representation of a device tilted with respect to an eye axis of a patient;

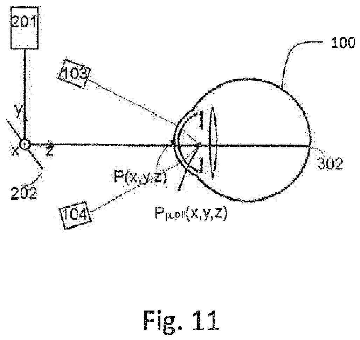

Fig. 11      shows a schematic cross-sectional side view of a setup used for determining a position of a pupil center of an examined eye;

Fig. 12      shows a schematic cross-sectional side view of a device for performing ophthalmic measurements on an eye according to the present disclosure, wherein the device comprises a topography unit and an optical coherence tomography unit; and

Fig. 13    shows a schematic cross-sectional side view of a device for performing ophthalmic measurements on an eye according to the present disclosure, wherein the device comprises a topography unit and an optical coherence tomography unit.

In the following, but without limitation thereto, specific details are expounded in order to give a full understanding of the present disclosure. It is clear to persons skilled in the art, however, that the present invention can be used in other embodiments, which can differ from the details expounded in the following.

In the above description and in the figures, the same reference numerals are used for corresponding features or units of different embodiments. However, the details expounded with regard to one of these features or units also hold accordingly for the features of other embodiments having the same reference sign. Further, the present invention is not limited to the concrete embodiments described above, which are merely examples how the present invention could be carried out. Other embodiments are possible, wherein it should be appreciated that features of one embodiment can be used in other embodiments as well.

The figures described in the following are schematic representations, which are not necessarily to scale, unless indicated otherwise.

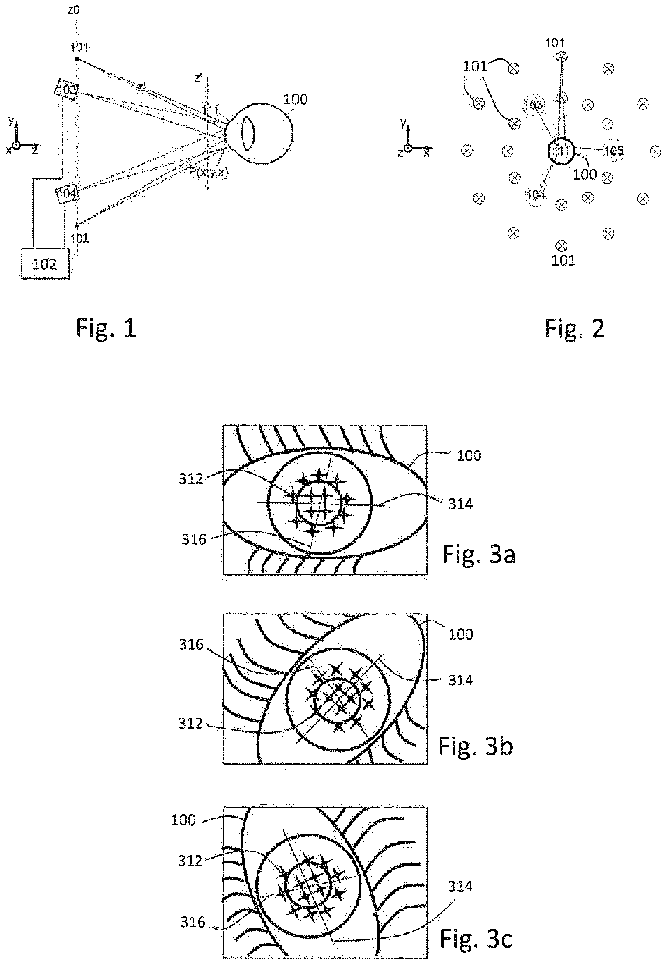

Figure 1 shows a cross-sectional side view of an embodiment of a device for performing ophthalmic measurements on an eye 100 according to the present disclosure. The device shown in Figure 1 corresponds to a basic configuration, which comprises a topographic unit for determining topographic features of the cornea 111 of an eye 100 under examination (in the following: "the eye" 100). Figure 1 shows a measurement position in which the device is positioned in front of the eye 100.

Figure 2 shows a cross-sectional front view of the device of Figure 1, wherein the cross-section is taken along the plane z0 indicated in Figure 1. As indicated in Figures 1 and 2, the x-axis corresponds to a horizontal axis. In the measurement position shown in Figure 1, the x-axis extends along a direction connecting the two eyes 100 of the patient. In an upright position of the patient, the x-direction lies in a horizontal plane. The $\gamma$-axis is orthogonal to the x-axis and represents a vertical direction. The z-axis is orthogonal to the x-axis and to the $\gamma$-axis and in the measurement position, the z-axis represents a direction along a line of sight of the eye 100. The plane z0, as well as a plane z' extend along an x-y-plane in front of the eye 100.

The device of the present embodiment comprises a plurality of first light sources 101 arranged along an inner circle (first circle) and an outer circle (second circle). In the device shown in Figures 1 and 2, the first light sources 101 are LEDs. Both the inner circle and the outer circle lie within the z0 plane and have the same center. In the measurement position, this center of the circles lies on a line of sight of the eye 100. In this case, the line of sight of the eye 100 corresponds to a center axis of the device. The device of Figures 1 and 2 comprises 12 LEDs 101 in an outer circle and 9 LEDs 101 in an inner circle. However, any other number and/or arrangement of first light sources 101 may be used. For example, a larger or smaller number of first light sources 101 may be provided in the inner and/or the outer circle. Further, the device may only comprise one circle or more than two circles of LEDs 101. Further, any type of light sources other than LEDs may be used for the first light sources 101, such as lasers. The first light sources 101 may also be represented by the outlets of a light guide or a plurality of light guides, such as optical fibers. Light of one or more light sources may be coupled into the light guide or into the plurality of light guides.

Rays emitted from each of the LEDs 101 are directed towards the eye 100 and reflected by the anterior cornea 111 of the eye 100. At least a part of the light beam which impinges onto the eye 100 is reflected by specular reflection, wherein an angle of the incident light is the same as an angle of the reflected light.

The reflected light beams are imaged onto three first optical detectors 103, 104, 105 arranged symmetrically around the center axis of the device. In the embodiment of Figures 1 and 2, three first optical detectors 103, 104, 105 are arranged on a circle around the center axis of the device, wherein the circle has the same center as the inner circle and outer circle of LEDs 101. However, the arrangement of first optical detectors 103, 104, 105 is not limited to this arrangement and also a larger or smaller number of first optical detectors may be provided. Further, the first optical detectors 103, 104, 105 do not need to be arranged in the plane z0 but may also be arranged in front or behind the plane z0, in which the LEDs 101 are arranged.

The first optical detectors 103, 104, 105 may comprise cameras including a two-dimensional image sensor, such as a CCD or CMOS sensor. Further, the first optical detectors 103, 104, 105 may comprise imaging optics, zooming optics, and/or focusing optics for imaging a desired portion of an object plane onto the corresponding sensor. Each optical detector 103, 104, 105 is configured to generate a two-dimensional image showing light spots resulting from each of the first light sources 101. In other words, each of the first light sources 101 generates a light spot in each of the generated two-dimensional images.

The first optical detectors 103, 104, 105 are connected to a controller 102, wherein the controller 102 is configured to process the two-dimensional pictures generated by the first optical detectors 103, 104, 105. In particular, the controller 102 is configured to determine centroid positions of the light spots of the two-dimensional images.

Figures 3a, 3b, and 3c each show a schematic representation of a two-dimensional image generated by the first optical detectors 103, 104, and 105, respectively. In the two-dimensional images of Figures 3a, 3b, and 3c, crosses 302 indicate detected positions of the light spots. For reasons of space, the schematic representation of Figures 3a, 3b, and 3c does not show all detected positions of light spots of the present embodiment. Further, a solid line 314 indicates a horizontal reference axis, which will be described later. A dashed line 316 indicates a keratometric cylinder axis resulting from an evaluation of topographic features performed by the controller 102.

In the following, the device of the embodiment of Figures 1 and 2 will be described again in detail.

Multiple point sources 101 (first light sources 101) are arranged symmetrically around the optical axis (z) of the device (ophthalmic apparatus), i.e., around the center axis of the device. Said point sources 101 direct light onto a cornea 111 of a subject's eye 100. A wavelength of the emitted light of the first light sources 101 is between 320-1500 nm. At least two cameras 103, 104, 105 (first optical detectors) are positioned at an off-axis position each consisting of a two-dimensional image sensor and a focusing lens, said cameras focused/centered on the z-axis of the ophthalmic apparatus at a plane z' found at a fixed distance (50-120 mm) in front of the ophthalmic apparatus. Said cameras 103, 104, 105 collect simultaneously light emanating from the multiple point sources 101 and reflected specularly from the anterior cornea 111.

The device of the present embodiment comprises a controller 102. The controller 102 comprises means for detecting centroid positions on the at least two two-dimensional image sensors 103, 104, 105 of the light collected from the multiple point sources 101 and reflected specularly from the cornea 111. The controller 102 further comprises means for modelling the optical configuration of the device and apply raytracing algorithms to calculate theoretical centroid positions. Further, the controller 102 comprises means for calculating theoretical centroid positions by raytracing light from the multiple point sources in said modelled optical configuration on a model cornea defined by topographic features and position with respect to the apparatus coordinate system. Said raytracing rays are specularly reflected on the model cornea and are traced back through the center of the aperture defined by the focusing lens onto the image plane, the position of the multiple rays from the multiple point sources on the image plane define the theoretical centroid positions.

Further, the controller 102 comprises means for calculating differences in position of detected and theoretically calculated light spot positions. The controller 102 further comprises means for minimizing said difference in position by adjusting topographic features and a position of the model cornea. The controller 102 is configured to determine topographic features of the anterior cornea 111 and a position of the eye 100 by finding a configuration of the model cornea that minimizes said difference. Topographic features of the anterior cornea 111 can include radius of curvature along steep axis, radius of curvature along flat axis, orientation of steep/flat axis, asphericity/conic constant of cornea along steep/flat axis, and/or higher order corneal aberrations. A position of the eye 100 is given by P(x,y,z) indicating coordinates of the corneal vertex with respect to a device-anchored coordinate system. When a patient is fixating on a target on the center axis of the device, this position of the eye (100) coincides with the intersection of the videokeratoscopic axis and the cornea. In alternative embodiments, the position of the eye (100) can be related to the intersection of any other eye axis (e.g., visual axis, line-of-sight, pupillary axis) and the anterior cornea. The different eye axes have for instance been described in the article "Mosquera, Samuel, Shwetabh Verma, and Colm McAlinden, "Centration Axis in Refractive Surgery." Eye and Vision 2.1 (2015): 4. Web".

In an alternative embodiment of the determination of topographic features of the cornea 111 and a position of the eye 100 with respect to the device, rays are traced from the detected spot position on each image sensor 103, 104, 105 through the aperture of the focusing lens onto the model cornea and reflected back onto the plane z0 containing the plurality of first light sources 101. The intersections of said rays with said plane z0 define the theoretical/simulated light source positions, i.e., positions of model light sources. A difference in position of the model light source positions with the actual light source positions is calculated. Said difference in position of model (theoretical) and actual light source position is minimized by changing the eye position and topographic features of the model cornea. The configuration that minimizes said difference determines the topographic features of the anterior cornea 111 and the eye position.

The reconstruction of the topographic features of the cornea 111 according to the embodiments described in this disclosure are not influenced by the eye position, as the eye position is taken into consideration in the reconstruction process. Thus, the measurements of topographic features are not influenced by the eye position. An eye position range where measurements of topographic features can be obtained is limited by the configuration of the first optical detectors 103, 104, 105 (magnification, sensor size) which determines the spatial range where spots are still imaged onto the sensors.

By using the above raytracing technique, it may be possible to reconstruct topographic features of the cornea 111 under consideration of the reconstructed position of the eye 100. This is possible even when the eye 100 is not centered with regard to an optical axis of the device and/or when the eye 100 has an arbitrary distance from the device.

The topographic features of the cornea 111 may be determined with regard to at least one reference axis of the eye 100, as explained below.

One topographic feature of the eye 100 is the axis 316 of the flat meridian (orthogonal to the axis of the steep meridian) or cylinder axis 316. Said cylinder axis 316 is commonly referenced with respect to a horizontal reference axis 314 at 0 deg, see Figures 3a, 3b, and 3c. The axis of cylinder of the refraction measurement is also referenced with respect to said horizontal reference axis 314.

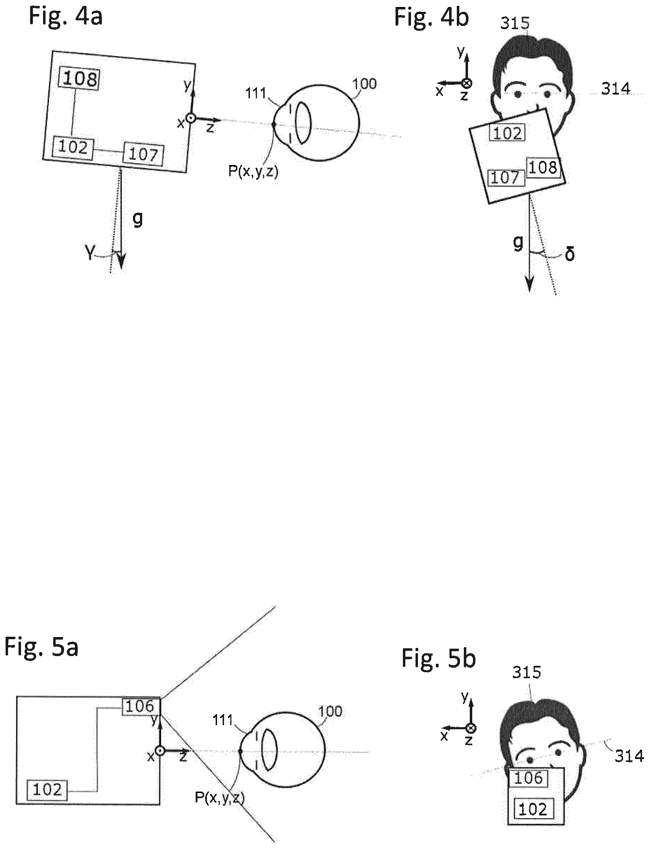

As shown, e.g., in Figures 4a and 4b, in one embodiment, which may be combined with any of the embodiments described herein, the horizontal reference axis 314 can be determined by measuring a patient 315 in an upright position and measuring simultaneously the orientation of the device by means of an orientation sensor 107 or accelerometer 107 of the device. Based on orientation information output by the orientation sensor 107 or the accelerometer 107, a horizontal reference axis 314 may be determined.

Said orientation sensor or accelerometer 107 can be controlled by the control unit 102 and output of the orientation sensor can be displayed to the user through a display unit 108 in order to guide the user in the alignment process when measuring a patient 315. For instance, when measuring a patient 315 in an upright position, the user should align the device such that the device coordinate system y axis is aligned with the g-force vector g of the gravitational field of the earth.

As shown in Figures 5a and 5b, in one embodiment, which may be combined with any of the embodiments described herein, the handheld device incorporates an additional image sensor (optical detector) 106 with a large field of view to capture an image of the face of the patient 315 or at least of an eye region of the face of the patient 315. On said image, the horizontal reference axis 314 is determined by the line joining the centers of the pupils of the patient's eyes.

In another embodiment, a reference axis is determined based on features detected on the images of the eye 100 obtained with the first optical detectors 103, 104, 105. Said features can include eye lid, iris shape and structure, pupil shape and stromal blood vessels.

Subsequent measurements of a cylinder axis on a same patient can/should be referenced with respect to a common reference axis. Said common reference axis is initialized in the first measurement according to any one of the aforementioned embodiments. Said common reference axis is referenced with respect to features of the eye 100 detected on the acquired images of each measurement. Said features can include iris shape and structure, pupil shape and stromal blood vessels.

Said common reference axis referenced with respect to structures of the eye has the advantage that cyclotorsional eye movements that occur between a supine and upright position of the head are taken into account when defining the cylinder axis 316 with respect to said common reference axis.

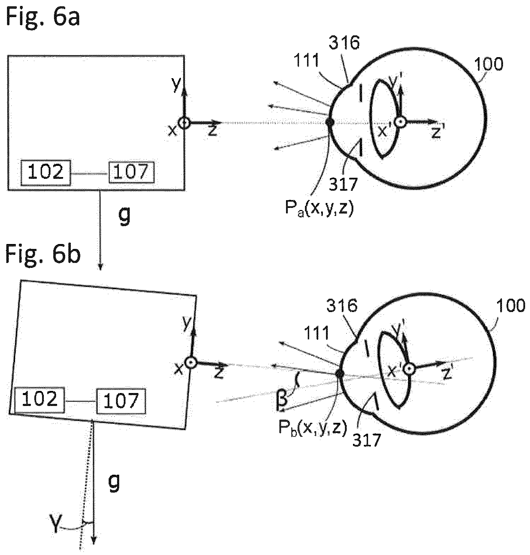

In Figures 6a and 6b it is shown that subsequent measurements of the eye 100 may differ in the alignment of an eye anchored coordinate system (x',y',z') with respect to a device coordinate system (x,y,z) (also referred to as device anchored coordinate system (x,y,z) or coordinate system fixed to the device) either because of eye movements of the patient or because of movements of the device. Generally, the alignment of coordinate systems (x',y',z') and (x,y,z) can be described by a transformation consisting of a translation vector t and a rotation matrix R such that $[x,y,z]=R*([x',y',z']+t_i)$ (matrix calculation).

Here the matrix R can be constructed by specifying a tilt around the x-axis, a tilt around the $\gamma$-axis and a tilt around the z-axis (cyclotorsional alignment). For instance, a tilt around the x-axis is described by the angle $\beta$ shown in Figure 6b. The translation vector t can be identified with the reconstructed position of the eye $P_i$. The eye z'-axis can be identified with any of the eye axes described in the literature such as the videokeratoscopic axis, the line-of-sight or the visual axis. For the topographic measurements, the videokeratoscopic axis is convenient, as this axis is aligned with the device z-axis (center axis) and goes through the reconstructed eye position P(x,y,z) if the patients is properly fixating a target on the center axis of the device. A priori, it is not known whether a patient properly fixated the target during the measurement. If subsequent measurements show a different alignment with respect to the device coordinate system, an average eye orientation should be determined from multiple measurements of eye alignments to identify an eye orientation corresponding to an orientation when a patient is fixating the target.

As a consequence of a tilt between two consecutive measurements, the reconstructed eye position P(x,y,z) in the device coordinate system does not always refer to the same location on the cornea 111. In order to reference subsequent measurements of topographic features of the eye to the same corneal location, first the alignment transformation $(R_i,t_i)$ specifying the transformation of the eye coordinate system $(x',y',z')_i$ and $(x',y',z')(i+1)$ between subsequent topographic measurements (i,i+1) should be determined.

The alignment transformation between subsequent topographic measurements can be determined by one or a combination of the following methods.

1. The alignment transformation is determined by aligning the surface normals of the reconstructed anterior corneal surface. The alignment transformation minimizes the alignment error between the reconstructed surface normal of subsequent measurements.

2. The alignment transformation is determined by reconstructing positions of scleral blood vessels and/or corneal limbus locations 316 visible in the images obtained with the first optical detectors 103, 104, 105. The position of said features with respect to the device coordinate system can be obtained by stereoscopic reconstruction using the position information of said features within each of the images obtained with the first optical detectors.

The alignment transformation is then identified as the transformation that maps the position of said features reconstructed in a first measurement to the position of said features reconstructed in the subsequent measurement.

3. The alignment transformation is determined by reconstructing positions of iris features including pupil boundary locations 317 visible in the images obtained with the first optical detectors 103,104,105. Reconstruction of positions of said features can be performed similarly to the reconstruction of the pupil center position (described later on). The alignment transformation is then identified as the transformation that maps the position of said features reconstructed in a first measurement to the position of said features reconstructed in the subsequent measurement.

To anchor the topographic features reconstructed from subsequent measurements with regard to the same eye coordinate system (x',y',z'), an average eye orientation/position with respect to the device coordinate system (x,y,z) is determined from the evaluated alignment transformations $(R_i,t_i)$. The average eye orientation corresponds to the alignment when a patient is properly fixating a target located on the center axis of the device.

Further, a new alignment transformation $(R_m,t_m)$. mapping the eye coordinate system of each measurement m to the average eye orientation/position can be determined using the previously determined alignment transformations $(R_i,t_i)$.

Said alignment transformations $(R_m,t_m)$ allow then to align subsequent topographic measurements and center with respect to the vertex of the average eye orientation.

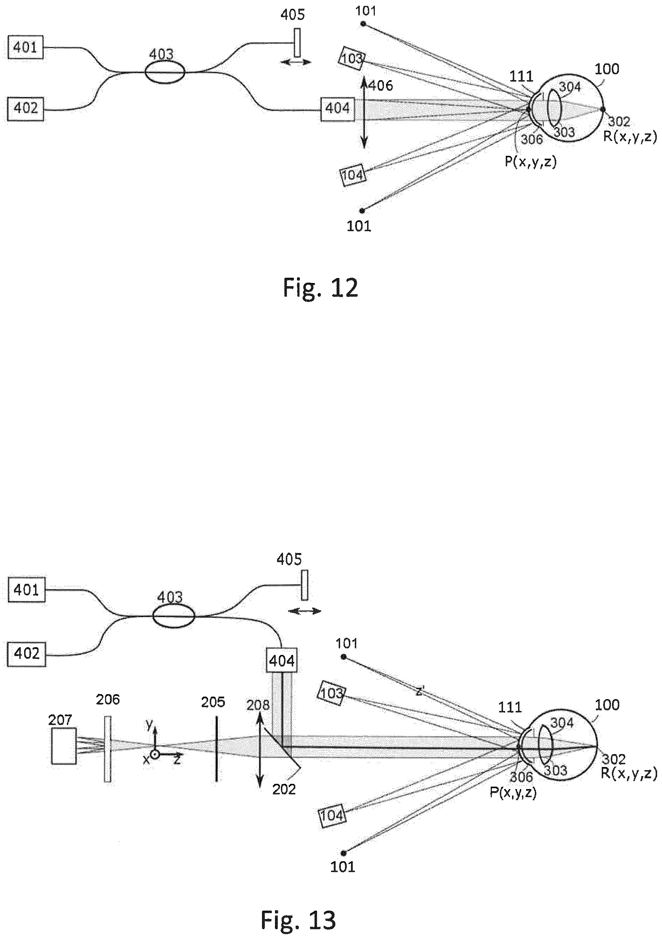

In the embodiment of the device shown in Figures 1 and 2, 12 LEDs 101 (A = 800 nm) are arranged in an outer circle (44 mm radius) and 9 LEDs 101 are arranged in an inner circle (25 mm radius) around the optical axis (center axis) of the device. Said LEDs 101 serve as light point sources (first light sources). Three camera units (first optical detectors) 103, 104, 105 comprising a CMOS image sensor and a focusing lens are arranged in a circle (radius 22 mm) around the optical axis (see Figure 2).

Figure 4 shows exemplary modelled raytracing spot positions as circles 402 and exemplary detected spot positions as crosses 404, for one of the first optical detectors 103, 104, 105. Each of the first optical detectors 103, 104, 105 generates a two-dimensional image comprising light spots. Based on these light spots, centroid positions (spot positions) can be extracted by the controller 102. For one of the two-dimensional images, Figure 4 shows the extracted spot positions as crosses 404. Further, a reconstruction procedure described below considers a model cornea and performs raytracing on said model cornea resulting in modelled raytracing spot positions shown as circles 402 in Figure 4. The reconstruction procedure tries to minimize a distance d between the modelled raytracing spot positions and the detected spot positions, such that a topography and a position of the model eye corresponds to a topography and a position of the real eye 100 as close as possible.

In other words, a topography and a position of the model cornea corresponds to a topography and a position of the real cornea 111 of the eye 100 as close as possible.

According to the present embodiment, the topographic features of the cornea 111 are reconstructed based on the flow chart scheme depicted in Figure 5, which provides an efficient computational implementation of the optimization problem. The model cornea is initialized with a fixed set of values for the topographic features of the cornea and a fixed value for the eye position. Said topographic features are radius of curvature along steep axis, radius of curvature along flat axis, orientation of steep/flat axis, asphericity/conic constant of cornea along steep/flat axis.

First, in step 502 the eye position of the model cornea is optimized by calculating position metrics from the detected centroid position that are strongly correlated with the eye position. The eye position of the model cornea is varied, until the position metrics evaluated from the traced, theoretical centroid positions equals position metrics from the detected centroid positions. The position metric of a position coordinate varies linearly with position (in first approximation). Hence, in one exemplary embodiment, only two raytracing calculations are needed to fit a position coordinate.

In a second step 504, the orientation of a keratometric cylinder axis 316 is determined by firstly fitting an ellipse to the detected centroids residing on the outer circle. This is done for the two-dimensional image of each camera (first optical detector) 103, 104, 105. The measured ellipse is defined by parameters $(a_m, b_m, \varphi_m)$, where $a_m$ denotes the short axis, $b_m$ denotes the long axis and $\varphi_m$ the angular orientation of the ellipse. Secondly, the measured ellipse is "normalized" by an ellipse $(a_n,b_n,\varphi_n)$ fitted to the traced, theoretical centroid spots for a cornea localized at the determined eye position, but having no cylinder.

Normalization is done by calculating the matrix P:

$$P = R(-\varphi_n) * \begin{bmatrix} 1/a_n & 0 \\ 0 & 1/b_n \end{bmatrix} * R(\varphi_n) * R(-\varphi_m) * \begin{bmatrix} a_m & 0 \\ 0 & b_m \end{bmatrix}.$$

Here $R(\alpha) = \begin{bmatrix} \cos(\alpha) & \sin(\alpha) \\ -\sin(\alpha) & \cos(\alpha) \end{bmatrix}$ denotes a two-dimensional rotation matrix. P is a matrix, obtained for each camera 103, 104, 105, that defines the cylinder of the anterior cornea by the relation

$$P = R(-\varphi_c) * \begin{bmatrix} a_c & 0 \\ 0 & b_c \end{bmatrix} * R(\varphi_c) .$$

Here $a_c/b_c$ gives the ratio of steep to flat radius, and $\varphi_c$ denotes the orientation of the cylinder axis.

To estimate $a_c$, $b_c$, $\varphi_c$ from the measured data, one can calculate a singular value decomposition of P to obtain unitary matrices U and V and a diagonal matrix S such that $P=U*S*_V'$. Here U is an orthogonal matrix that approximates R $(-\varphi_c)$ and the diagonal elements of S approximate $a_c$, $b_c$.

This calculation is performed for each camera 103, 104, 105. Hence, the estimated cylinder is an average value of the 3 cylinder measurements for each camera 103, 104, 105.

As a third step 506 the curvature of steep and flat axis is estimated for a fixed conic constant of the model cornea. Here the curvature metric is the aforementioned matrix S (averaged over 3 measurements/cameras) evaluated for both the detected centroid spot positions 404 and the raytraced, theoretical centroid spot positions 402. The curvature metric varies linearly in first approximation. Hence, according to one exemplary embodiment, only two raytracing calculations are required to fit steep/flat curvature.

As a fourth step 508 the conic constants along steep/flat axis are estimated. This is done by repeating the third step 505 for different values of the conic constants. As a metric, the difference d in position of detected and theoretically calculated positions is taken. The conic constants are determined by minimizing the metric (distance d).

Steps 502, 504, 506, and 508 can be iterated n-times to improve the accuracy of the found solution. Typically, n may be predetermined to be a fixed number, e.g. n = 2. Steps 502, 504, 506, and 508 may be performed by the controller 102.

Figure 6 shows an embodiment of a device according to the present disclosure comprising a topography unit and a wavefront unit. The topography unit may correspond to the topography unit of the device described with regard to Figures 1 to 5 above. In particular, an arrangement and properties of the first light sources 101 and the first optical detectors 103, 104, 105 may be the same as described with regard to Figures 1 to 5 above.

The wavefront unit of the embodiment shown in Figure 6 comprises a light source 201 (second light source) emitting collimated light towards a beam splitter 202. The light source may emit light, e.g., having a wavelength between 400 nm and 1100 nm. The collimated light is directed towards the eye 100 along the z-direction. The collimated light generates an illumination spot at the retina 302 of the eye 100. This illumination spot serves as a point source for a wavefront propagating in negative z-direction and exiting the eye 100 through the cornea 111. If the eye 100 had perfect optics, the wavefront exiting the eye 100 would correspond to a plane wave. However, due to internal aberrations of the eye (e.g., higher order aberrations and lower order aberrations), the exiting wavefront is aberrated. Higher order aberrations may occur, e.g., due to irregularities of the lens 304 or the cornea 111 of the eye 100 or misalignments of the lens (304) and/or the cornea (111) with respect to a visual axis.

The wavefront propagates through the beam splitter 202 and through relay lenses 203, 204. Relay lenses 203 and 204 are in confocal arrangement, i.e., a focal point of relay lens 203 corresponds to a focal point of relay lens 204. The relay lenses 203, 204 image a plane z', which is located at a fixed distance (e.g., 50 mm to 120 mm) in front of the device, onto a plane of a lenslet array 206. In other words, the plane of the lenslet array 206 is optically conjugate with the plane z'.

Further, a bandpass filter 205 is provided between the relay lenses 203, 204. The bandpass filter 205 may also be provided in front or behind the pair of relay lenses 203, 204. The bandpass filter 205 is configured to block light emitted from the first light sources 101 of the topography unit and to transmit light from the light source 201.

The wavefront impinges on the lenslet array 206, which is part of a typical Hartmann-Shack wavefront sensor. The wavefront sensor further comprises an image sensor (second optical detector) 207. The lenslet array 206 comprises a plurality of lenslets each having a focal length $f_L$.

The wavefront sensor comprises a controller which may be the controller 102 used for the topography unit (not shown in Figure 6). The controller of the wavefront sensor is configured to detect centroid positions on the image sensor 207 of the light focused by the lenslets (microlenses) of the lenslet array. The controller of the wavefront sensor is further configured to reconstruct the wavefront at the plane containing the lenslet array 206 from the detected centroid positions, said plane being conjugate with the plane z' found at a fixed distance in front of the device. More precisely, the controller of the wavefront sensor reconstructs the wavefront based on shifts between known ideal centroid positions of a plane wavefront and the centroid positions generated on the image sensor 207 by the aberrated wavefront.

Said wavefront aberrations are described by Zernike coefficients of radial order 0 until at least radial order 4.

In the present embodiment, the information regarding the position of the eye 100 determined by the topography unit is used by the wavefront unit in the following way. Aberrations of the wavefront at a plane having a fixed distance from the eye 100 (second plane) are measured by backpropagating the wavefront measured at the sample plane z' to the plane having a fixed distance from the eye 100. A propagation distance used for the backpropagating is derived from the previously or simultaneously measured position of the eye 100 P(x,y,z) (position of the cornea 111 of the eye 100). The plane having a fixed distance from the eye 100 may be a plane having a predetermined distance in the z-direction of zero or larger from the measured position P(x,y,z) of the cornea 111. Further, the plane can be positioned left or right of the measured position P(x,y,z) of the cornea 111 in the representation of Figure 6. For example, the plane having a fixed distance from the eye 100 may be a pupil plane of the eye 100 or a spectacle plane of the eye 100.

The propagation (i.e., the backpropagation) of the wavefront may be calculated by the method of angular spectrum propagation as described in Goodman, J. W. (1996) "Introduction to Fourier Optics", McGraw-Hill Series in Electrical and Computer Engineering. The propagation as well as the other computational steps described above may be carried out by a suitable controller of the wavefront unit. The controller of the wavefront unit may be the same controller 102 as for the topography unit (see Figure 1).

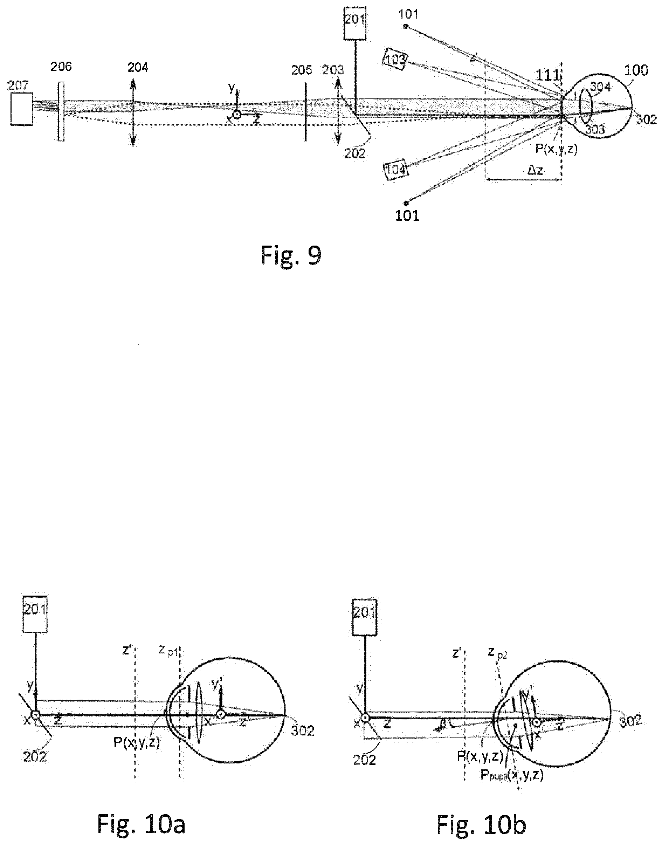

Further compensations of the measured wavefront related to the eye alignment can be performed. As shown in Figures 10a and 10b, as for the topographic measurements, subsequent wavefront measurements of the eye may differ in the alignment of an eye anchored coordinate system (x',y',z') with respect to a device coordinate system (x,y,z). From a set of simultaneously acquired topographic measurements an average eye orientation with respect to the device coordinate system can be determined and an alignment transformation $(R_m, t_m)$ mapping the eye coordinate system of each measurement m to the average eye orientation/position can be evaluated. These alignment transformations (Rm,tm) can be aimed at compensating misalignments between subsequent wavefront measurements.

The device allows to calculate intraoperative refraction/ocular aberrations when assuming preoperative corneal shape.

According to one embodiment the device allows to calculate internal aberrations $W_i$ of the eye. Internal aberrations $W_i$ of the eye 100 are calculated by subtracting corneal aberrations $W_c$ from the total ocular aberrations $W_{tot}$ obtained from the wavefront measurement, i.e. $W_i = W_{tot} - W_c$. Corneal aberrations $W_c(x,y)$ are related to a corneal height profile $z_c(x,y)$ of the cornea 111 as $W_c(x,y) = (n_c-1)*z_c(x,y)*2*pi/\lambda$, where $n_c$ denotes the refractive index of the cornea 111, and $\lambda$ the wavelength of the light source 201.

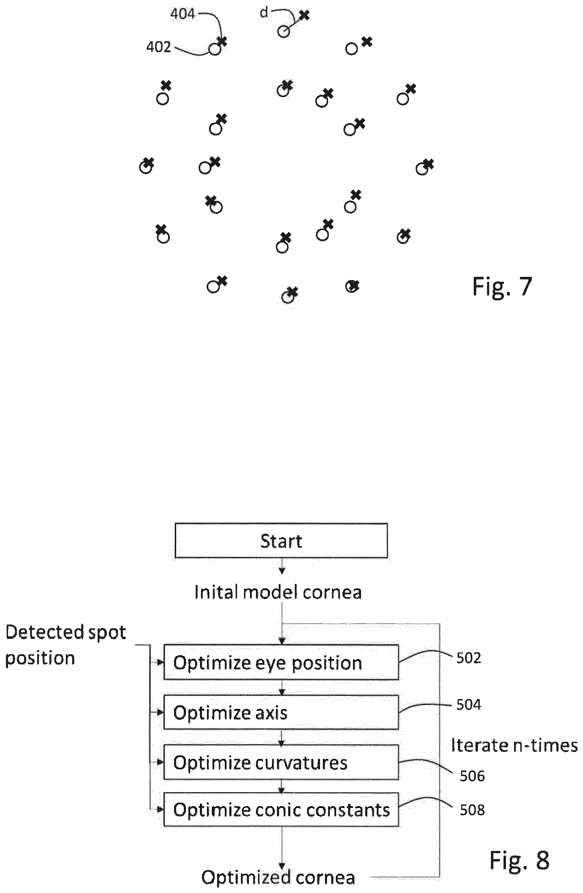

Here ocular and corneal aberrations are calculated with respect to the center of the pupil $P_{pupil}(x,y)$ which can differ from the reconstructed, lateral eye position P(x,y) related to the corneal vertex P(x,y,z). The pupil center position $P_{pupil}(x,y)$ can be inferred from the pupil center positions evaluated on the multiple cameras 103, 104, 105 of the topography unit, see Figure 8.

Figure 8 shows how a pupil center $P_{pupil}(x,y,z)$ of the eye 100 is viewed on the multiple cameras (first optical detectors) 103, 104, 105 of the topography unit. To determine the position $P_{pupil}(x,y,z)$ rays are traced from the detected center points of the pupil on the two-dimensional images of the cameras 103, 104, 105 onto the reconstructed anterior cornea (model cornea), where rays are refracted onto the common intersection point $P_{pupil}(x,y,z)$. The raytracing model may incorporate a posterior corneal surface located at an average central corneal thickness (CCT), e.g. 0.5 mm, distance behind the anterior surface. Further, the posterior radii of curvature may be having a fixed ratio (e.g. 0.84) to the reconstructed anterior radii of curvature. Further, a typical refractive index of the cornea at the light source (101) wavelength may be assumed.

If the backtraced rays do not intersect in a common point, the position $P_{pupil}(x,y,z)$ can, for instance, be estimated by taking the average x,y position of the backtraced ray positions at a distance z that minimizes the contour length of the line segments connecting the multiple ray positions within the anterior segment of the eye.

The eye 100 is fixated on the collimated light source 201.

Similarly, any other position of a feature of the eye detected in the multiple cameras (first optical detectors) and located within the anterior segment of the eye can be reconstructed.

Figure 9 shows an embodiment of a device according to the present disclosure comprising a topography unit and an optical coherence tomography (OCT) unit. The OCT unit is an OCT measurement unit for the measurement of intraocular distances (distances between surfaces within the eye 100). The OCT unit comprises a light source 401 (third light source), a photodetector 402 (third optical detector) at a detector arm of the OCT unit, a light coupler 403, an object beam 404 (object arm) directed on the eye 100 and focused on the retina 302, a tunable lens 406, and a reference beam (reference arm) directed on a reference mirror 405. The topography unit of the present embodiment may be the same or a similar topography unit as the topography unit described with regard to Figures 1 to 5. The tunable lens 406 is optional.

Figure 9 depicts an embodiment of a handheld device that includes an optical coherence tomography (OCT) unit allowing to measure positions of intraocular surfaces combined with a topographic unit allowing to simultaneously measure the corneal vertex position P(x,y,z) of the eye 100.

The optical coherence tomography (OCT) unit is configured to measure a retinal position R(x,y,z). The OCT unit of the device comprises four arms that each may comprise a respective light guide for directing the light generated by the light source 401 and/or reflected back from the eye 100. The OCT unit comprises a light source arm comprising the light source 401, a detector arm comprising the optical detector 402, an object arm configured to generate the object beam 404, and a reference arm comprising the reference mirror 405. Further, a light coupler 403 is arranged at the intersection of the four arms.

At the object arm an object beam 404 is generated and light is transmitted onto a patient's eye 100. At the reference arm, a reference beam is generated and light is directed onto a reference mirror 405. Reflected light from the sample (the retina 302 of the eye 100) and from the reference mirror 405 interferes at the detector 402. Here the OCT unit can be any of the following: Spectral Domain OCT using a broadband source or a swept source, or time domain OCT.

According to the present embodiment, the reference mirror 405 can be adjusted to control an optical pathlength in the reference arm. Said optical pathlength may be adjusted according to the measured corneal vertex position P(x,y,z) (position of the eye 100) obtained from the topography unit. The optical pathlength may further be adjusted according to an expected position of an intraocular surface of the eye 100 from which an interference signal should be collected. Said adjustement allows to reduce the scan depth of single A-scan, relaxing thereby requirements on the used light source and detection scheme.

An intraocular surface can be, e.g., the posterior corneal surface 306, the anterior lens surface 303, the posterior lens surface 304 or the retina 302. Based on the collected A-scan signal, an intraocular position can be inferred with respect to a device-anchored coordinate system. Simultaneously the vertex position P(x,y,z) is measured with the topography measurement. From the two measurements an intraocular distance can be evaluated.

According to an embodiment, light emitted into the object arm 404 can be focused via a tunable lens 406 onto an ocular surface of the eye 111, 306, 303, 304, 302 in order to maximize the signal-to-noise ratio of the signal collected from the ocular surface. The focal distance of the tunable lens 406 is adjusted according to the measured corneal vertex position P(x,y,z) (position of the eye 100) obtained from the topography unit. The focal distance of the tunable lens 406 is further adjusted according to an expected position of an ocular surface of the eye 100 from which an interference signal should be collected.

For instance, an axial length (a distance between the central anterior cornea 111 and the retina 302) can then be evaluated from the simultaneously measured retinal apex position R(x,y,z) and corneal vertex position P(x,y,z). If the corneal vertex is not aligned with the device optical axis (center axis) the axial length measurement may be corrected for the off-axis position. This correction can be achieved by taking the corneal topography measurement of the topography unit into consideration.

Figure 10 shows an embodiment of a device according to the present disclosure comprising a topography unit, a wavefront unit, and an optical coherence tomography (OCT) unit. Each of these units may be identical or at least similar to the respective units described with regard to the aforementioned embodiments of Figures 1 to 9. Therefore, a detailed explanation of overlapping features is omitted.

In the embodiment of Figure 10, the OCT unit and the wavefront unit use the same light source 401. Figure 10 depicts an embodiment of a handheld device integrating a corneal topography measurement, a wavefront and an intraocular distance measurement of an eye 100. According to this embodiment, a common light source 401 is used for both the wavefront unit and the OCT unit. The remaining features of the present embodiment, in particular the features of the topography unit, the wavefront unit, and the OCT unit may be identical to the features of these units of any of the embodiments described above.

It should be noted that the embodiments described above can be combined with each other in any suitable way. In particular, the above embodiments can be combined to one device comprising a topography unit and, additionally, a wavefront unit and/or an OCT unit. All or some of the features and advantageous of these units may individually apply to such a combined device.

In view of the above, aspects of the present disclosure are concerned with a technique for performing ophthalmic measurements on an eye 100. The device comprises a topography unit and, if necessary, additional units for measuring different properties of the eye 100. These additional units may comprise, e.g., a wavefront unit and an OCT unit. The topographic unit is configured to determine topographic features of the cornea and a position of the eye with respect to the device. The other units, if present, may use the topographic features and/or the position of the eye for performing further calculations and/or evaluations. Each of the units may comprise a controller for performing the respective computational steps or a centralized controller 102 may be provided, which controls the entire device.

The above technique provides a handheld, non-contact ophthalmic apparatus allowing for measurements not only during the surgical intervention when the patient is in a supine position, but also during a pre-or postoperative examination when the patient is in an upright position.

Apart from the measurement of the eye's wavefront aberration, the technique provides a measurement of the anterior corneal topography when assessing the outcome of cataract surgery or when planning implantation of aspheric or toric IOLs. By using the aforementioned topography unit, the measurement with the handheld apparatus is independent of the relative eye position since the eye position is measured simultaneously with respect to the apparatus and reconstruction algorithms of the eye's corneal topography and wavefront that take into account the eye position are applied.

The technique according to this disclosure provides a non-contact measurement of the eye's wavefront aberrations and an eye's anterior corneal topography and/or intraocular distances using a handheld device. The technique further provides a measurement of an eye position with respect to the handheld device and applies reconstruction algorithms that render the measurement of the eye's corneal topography, wavefront, and/or intraocular distances independent from the relative position of the eye with respect to the device.

**Claims**

1. A device for performing ophthalmic measurements on an eye (100), comprising:

   a plurality of first light sources (101) each configured to emit light towards a cornea (111) of the eye (100);
   a plurality of first optical detectors (103, 104, 105) each configured to generate a two-dimensional image of a plurality of light spots (312) each resulting from light emitted by one of the plurality of first light sources (101) and reflected by the cornea (111) towards the corresponding first optical detector (103, 104, 105); and
   a controller (102) configured to determine topographic features of the cornea (111) and a position of the eye (100) with respect to the device by performing raytracing on a modelled optical configuration and by comparing results of the raytracing with positions of the plurality of first light sources (101) and/or with positions of the light spots (312) in the two-dimensional images.

2. The device of claim 1, wherein the controller (102) is configured to
   consider a model cornea as part of the modelled optical configuration;
   perform raytracing of light rays emitted by model light sources and reflected by the model cornea;
   compare results of the raytracing with positions of the plurality of first light sources (101) and/or with positions of the light spots (312) in at least one of the two-dimensional images;
   adjust topographic features of the model cornea and/or a position of the model cornea;
   repeat the steps of considering, performing, and comparing; and
   determine the topographic features of the cornea (111) and the position of the eye (100) based on the topographic features of the model cornea and the position of the model cornea.

3. The device of claim 2, wherein the controller (102) is configured to
   perform the raytracing by raytracing of light rays emitted at known positions of the plurality of first light sources (101);
   compare positions of the raytraced light rays on a model optical detector with measured positions of the light spots (312) in at least one of the two-dimensional images.

4. The device of claim 2, wherein the controller (102) is configured to
   perform the raytracing by raytracing of light rays back from measured positions of the light spots (312) towards the model cornea;
   compare positions of model light sources determined by the ray tracing with known positions of the first light sources (101).

5. The device of any of claims 1 to 4, wherein
   the plurality of first light sources (101) is arranged at predetermined positions with regard to a center axis of the device, and, optionally,
   each of the plurality of first optical detectors (103, 104, 105) is arranged at a location away from the center axis of the device.

6. The device of claim 5, wherein
   the plurality of first light sources (101) comprises light sources (101) arranged along a first circle and an axis extending through the center of the first circle and being orthogonal to the first circle is defined as the center axis of the device, and, optionally,
   the plurality of first light sources (101) comprises light sources (101) arranged along a second circle having the same center as the first circle and a different radius than the first circle.

7. The device of any of claims 1 to 6, wherein
   the topographic features of the cornea (111) comprise at least one feature selected from the list of radius along a steep axis, curvature along a steep axis, asphericity along a steep axis, radius along a flat axis, curvature along a flat axis, asphericity along a flat axis, orientation of a steep axis with regard to a reference axis of the eye (100), orientation of a flat axis with regard to a reference axis of the eye (100), radius along a horizontal axis, curvature along a horizontal axis, asphericity along a horizontal axis, radius along a vertical axis, curvature along a vertical axis, asphericity along a vertical axis, and higher order corneal aberrations, and/or
wherein the position of the eye (100) with respect to the device is represented by coordinates of the intersection of a reference axis of the eye (100) and the anterior corneal surface of the eye (100) with respect to a coordinate system fixed to the device.

8. The device of any of claims 1 to 7, further comprising:

   a second light source (201) for illuminating the eye (100) with light so as to produce a wavefront that propagates along an optical path; and
   a wavefront sensor (206, 207) configured to provide a measure indicative of aberrations of the eye (100).

9. The device of claim 8, wherein the controller (102) is configured to
determine wavefront aberrations with regard to a first plane (z'), wherein the first plane (z') has a fixed distance from the device; and
determine wavefront aberrations of the eye (100) with regard to a second plane having a predetermined position with regard to the position of the eye (100) by backpropagating the determined wavefront from the first plane (z') to the second plane, based on the measured position of the eye (100) with respect to the device.

10. The device of any of claim 8 or 9, wherein
the wavefront sensor (206, 207) comprises a two-dimensional lenslet array (206) and a second optical detector (207) for generating a two-dimensional image of light spots generated by lenslets of the lenslet array (206) by focusing the wavefront onto the second optical detector (207).

11. The device of any of claims 8 to 10, wherein the controller (102) is configured to
determine an average eye orientation with respect to a device anchored coordinate system from a set of topographic measurements obtained from the first optical detectors (103, 104, 105); and
align the set of topographic measurements with respect to the average eye orientation by means of evaluated alignment transformations.

12. The device of claim 11, wherein
the controller (102) is configured to align a set of wavefront measurements performed by the wavefront sensor (206, 207) with respect to an eye anchored coordinate system by means of said alignment transformations.

13. The device of any of claims 8 to 12, wherein
the controller (102) is configured to calculate internal aberrations of the eye (100) by subtracting corneal aberrations of the eye (100) from total aberrations of the eye (100), wherein the corneal aberrations are based on the determined topographic features of the eye (100) and the total aberrations are based on the measure indicative of aberrations of the eye (100) provided by the wavefront sensor (206, 207).

14. The device of any of claims 1 to 13, further comprising:

   an optical coherence tomography unit for determining intra-ocular distances comprising:

      a light coupler (403);
      a third light source (401) configured to emit light towards the light coupler (403);
      a reference arm comprising an adjustable reference mirror (405);
      an object arm configured to generate an object beam (404) for directing light generated by the third light source (401) towards the eye (100); and
      a detector arm comprising a third optical detector (402), wherein

   the light coupler (403) is configured to couple part of the light generated by the third light source (401) into the reference arm and to couple part of the light generated by the third light source (401) into the object arm; and
   the light coupler (403) is configured to couple light reflected by the eye (100) into the detector arm and to couple light reflected by the adjustable reference mirror (405) into the detector arm, such that the light reflected by the eye (100) and the light reflected by the adjustable reference mirror (405) interfere at the third optical detector (402).

15. The device of claims 8 and 14, wherein

the third light source (401) of the optical coherence tomography unit serves as the second light source.

16. The device of claim 14 or 15, wherein
the controller is configured to set an initial position of the adjustable reference mirror (405) based on the determined position of the eye (100), and/or
wherein the device comprises a tunable lens (406) within the object beam (404) and wherein the controller is configured to set an initial focal length of the tunable lens (406) based on the determined position of the eye (100).

17. A method for performing ophthalmic measurements on an eye (100) with a device, comprising:

emitting light towards a cornea (111) of the eye (100) by a plurality of first light sources (101) of the device;
generating, by a plurality of first optical detectors (103, 104, 105) of the device, a plurality of two-dimensional images of a plurality of light spots (312) each resulting from light emitted by one of the plurality of first light sources (101) and reflected by the cornea (111) towards the corresponding first optical detector (103, 104, 105); and
determining topographic features of the cornea (111) and a position of the eye (100) with respect to the device by performing raytracing on a modelled optical configuration and by comparing results of the raytracing with positions of the plurality of first light sources (101) and/or with positions of the light spots (312) in the two-dimensional images.

Fig. 1

Fig. 2

Fig. 3a

Fig. 3b

Fig. 3c

Fig. 4a

Fig. 4b

Fig. 5a

Fig. 5b

## Fig. 6a

## Fig. 6b

404 —

d —

402 —

Fig. 7

Start

Inital model cornea

Detected spot position

Optimize eye position — 502

Optimize axis — 504

Iterate n-times

Optimize curvatures — 506

Optimize conic constants — 508

Optimized cornea

Fig. 8

Fig. 9

Fig. 10a          Fig. 10b

Fig. 11

Fig. 12

Fig. 13

Europäisches
Patentamt

European
Patent Office

Office européen
des brevets

## EUROPEAN SEARCH REPORT

Application Number

EP 16 19 8190

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2016/150952 A1 (RAYMOND THOMAS D [US] ET AL) 2 June 2016 (2016-06-02)<br>* abstract; figures 16, 18,19A,19B *<br>* paragraphs [0115], [0116], [0165], [0176], [0196], [0200] - [0207], [0212], [0238] * | 1-14,16, 17<br>15 | INV.<br>A61B3/107<br>A61B3/00<br>G06F19/00 |
| A | US 2015/190046 A1 (DE PAZ SICAM VICTOR ARNI [NL] ET AL) 9 July 2015 (2015-07-09)<br>* abstract; figures 1-3,13 *<br>* paragraphs [0023] - [0031] *<br>* paragraphs [0115] - [0119] *<br>* paragraphs [0208] - [0211] * | 1-17 | |

TECHNICAL FIELDS
SEARCHED     (IPC)

A61B
G06F

The present search report has been drawn up for all claims

| Place of search | Date of completion of the search | Examiner |
|---|---|---|
| Munich | 12 May 2017 | Daniel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

**EP 3 320 830 A1**

## ANNEX TO THE EUROPEAN SEARCH REPORT
## ON EUROPEAN PATENT APPLICATION NO.

EP 16 19 8190

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2017

| Patent document cited in search report | Publication date | Patent family member(s) | Publication date |
|---|---|---|---|
| US 2016150952 A1 | 02-06-2016 | NONE | |
| US 2015190046 A1 | 09-07-2015 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8403919 B2

**Non-patent literature cited in the description**

- Introduction to Fourier Optics. **GOODMAN, J. W.** Electrical and Computer Engineering. McGraw-Hill Series, 1996

- **MOSQUERA ; SAMUEL ; SHWETABH VERMA ; COLM MCALINDEN.** *Centration Axis in Refractive Surgery,* 2015